# EUROPEAN PATENT APPLICATION

(11) **EP 3 321 296 A1**
(43) Date of publication of application: **16.05.2018**
(21) Application number: 16820741.3
(22) Date of filing: 22.06.2016
(51) Int. Cl.: C08F 218/08, C08F 212/36, C08F 212/08, A61K 47/30

(54) **MICRO-NANO MEDICAL ADSORBENT RESIN POWDER MATERIAL**

(30) Priority: 09.07.2015 CN 201510399777; 25.07.2015 CN 201510441375; 19.08.2015 CN 201510510311; 19.08.2015 CN 201510510251; 16.01.2016 CN 201610028904; 25.01.2016 CN 201610047350; 27.02.2016 CN 201610109578
(71) Applicant: Yu, Jie, Beijing 100012 (CN)
(72) Inventor: Yu, Jie, Beijing 100012 (CN)
(74) Representative: Lang, Christian
(86) International application number: PCT/CN2016/086677
(87) International publication number: WO 2017/005090

(57) **Abstract**

A micro-nano medical adsorbent resin powder material, characterized in that: it is a high-molecular polymer including carbon ions, hydrogen ions, and oxygen ions.

## Description

### Background

In the past, the material shape of the macroporous adsorption resin for medical use was in a regular granular form, and the macroporous adsorption resin was used as an adsorbent filled in the hemoper fusion device in the blood purification therapy. The range of the particle size is 80 mesh (178 µm). No medical resin with micro-nano particle size has been reported in the literature.

### Summary of the invention

The objectives of the present invention are to provide a technical solution of a medical absorbent resin material having a micro-nano particle size and a technical solution of using the material.

The contents of the present invention are as follows: A micro-nano medical adsorbent resin powder material, characterized in that: it is a high-molecular polymer including carbon ions, hydrogen ions, and oxygen ions; the molecular structure includes a benzene ring and a hydroxyl group, and the polymer includes at least two or more molecules; the overall particle size is in a range of 0.01-50 micrometer (um), preferably 0.01-1.5 micrometer (10-1500 nm).

### Detailed description of the invention

Embodiments of the present invention:
Embodiment 1: The polymer is a polymer including vinyl acetate and divinyl-benzene.
Embodiment 2: The polymer is a polymer including styrene and divinyl-benzene.
Embodiment 3: A carbohydrate chains is bound to a binding site of a carbon skeleton and/or a branched-chain of the polymer to form a glycosylated polymer.
Embodiment 4: Transition- metal ions are embedded in the carbon skeleton of the polymer; and the metal element is iron element.

Embodiments of the use of the micro-nano medical absorbent resin powder material of the present invention:
Embodiment 5: The powder material is used as a coating agent of skin, body tissue, living organ or a surface of living organism.
Embodiment 6: The powder material is used as a vector carrying a drug or a life-intervention substance, including a stem cell.
Embodiment 7: The powder material is used as a medical molding material, including a bulking agent for a hollow fiber, a silicone blood transfusion tube, a silicone infusion tube, a dialysis membrane, or a medical coating, or a shaping material integrated with a surface of the hollow fiber, the silicone blood transfusion tube, the silicone infusion tube, the dialysis membrane, or the medical coating.
Embodiment 8: The powder material is used as a solute, and an injection solution or a physiological saline or ethanol having different purities, including fruit wine is used as a solvent to prepare a solution.
Embodiment 9: The powder material is added into a shaping material to make a paste, or a pill, or a grease agent.

In the above-mentioned embodiments 7 to 9, the powder material of the present invention is added to a molding material or a shaping material or a solution, in which the maximum proportion of the powder material is limited by the maximum powder material that can be added, and the minimum proportion of the powder material is not limited. Under the conditions, without affecting the use of molding material or shaping material or solution, the amount of added powder material determines the effect.

The micro-nano effects and quantum effects of the material of the present invention are shown as follows: In the micro-nano material interface microenvironment, micro-nano particles of the adsorption resin can penetrate the skin, muscle tissue and cell membrane, and can have collisions with affinitive adsorption materials actively driven by equilibrium potential, so that the adsorption materials, including active cells, intracellular genetic material DNA\RNA, and apoptotic macromolecules of life substance decompose and break down, and the cell debris are affinitive-absorbed into the metabolic system and excreted. Animal model tests have proven that the coating of micro-nano resin powder material on the blood vessel wall can make the fatty substances stacked on the inner wall of the blood vessel decompose and flow away with blood like a "smoke", and make the cancerous tissues instantly breakdown into a "smoke" and flow away with blood. The volunteer is a liver cancer patient who has reached the state of clinical liver necrosis (a death notice has been received). The liver was exposed in the surgery. The micro-nano resin powder material was directly applied on the outside of the liver, such that the material rapidly penetrated into the liver tissue, and then the liver tissue began to restore blood color. In the blood drawn from the hepatic vein, cancerous cell tissue molecular debris were observed. After the liver restored blood color and the color of the drawn blood recovered normally, abdominal cavity was sutured. The whole period of the surgery was four hours. Three days later, the volunteer returned to normal diet and left hospital after one week. About a year before this case, a liver transplantation surgery was made. Because of the donor necrosis, the bad liver removed from the patient was forced to be put and immersed into the physiological saline solution of micro-nano resin powder material. Necrotic tissues made the solution turn black, while the liver in vitro restored blood color and was then implanted in the body. Without any postoperative adverse reactions, the patient soon left the hospital. In addition, the inventor and his colleagues volunteered for many trials. The effects are: oral administration can adjust the blood sugar, coating on skin can achieve whitening, eliminate subcutaneous hydrops, and quickly heal wounds and so on.

The polymer molecule of the medical absorbent resin powder material of the present invention is a chemical synthetic product, which has chemical stability, does not participate in a biochemical reaction, and has no side effect in clinical use.

Various use embodiments of the present invention are different use forms of the resin powder of the present invention, which are respectively suitable for skin coating, oral administration, injection dosage, etc.

Substantive distinguishing features of the present invention are to show the micro-nano effect and quantum effect of the medical absorbent resins.

A significant improvement provided by the present invention opened a new application field of the medical absorbent resins.

## Claims

1. A micro-nano medical adsorbent resin powder material, **characterized in that**: the powder material is a high-molecular-weight polymer including carbon ions, hydrogen ions, and oxygen ions; a molecular structure includes a benzene ring and a hydroxyl group, and the polymer includes at least two or more molecules; an overall particle size is in a range of 0.01-50 micrometer (um), preferably 0.01-1.5 micrometer (10-1500 nm).

2. The micro-nano medical adsorbent resin powder material according to claim 1, **characterized in that**: the polymer is a polymer including vinyl acetate and divinyl-benzene.

3. The micro-nano medical adsorbent resin powder material according to claim 1, **characterized in that**: the polymer is a polymer including styrene and divinyl-benzene.

4. The micro-nano medical adsorbent resin powder material according to claim 2 or 3, **characterized in that**: a carbohydrate chains is bound to a binding site of a carbon skeleton and / or a branched-chain of the polymer to form a glycosylated polymer.

5. The micro-nano medical adsorbent resin powder material according to claim 2 or 3, **characterized in that**: transition-metal ions are embedded in the carbon skeleton of the polymer.

6. The micro-nano medical adsorbent resin powder material according to claim 2 or 3, **characterized in that**: transition-metal ions are embedded in the carbon skeleton of the polymer; and the metal is iron.

7. A use of the micro-nano medical adsorbent resin powder material according to any one of claims 1, 2, 3, 4, 5 or 6, **characterized in that**: the powder material is used as a coating agent a surface of skin, a body tissue, a living organ or a living organism.

8. A use of the micro-nano medical adsorbent resin powder material according to any one of claims 1, 2, 3, 4, 5 or 6, **characterized in that**: the powder material is used as a vector carrying a drug or a life-intervention substance, including a stem cell.

9. A use of the micro-nano medical adsorbent resin powder material according to any one of claims 1, 2, 3, 4, 5 or 6, **characterized in that**: the powder material is used as a medical molding material, including a bulking agent for a hollow fiber, a silicone blood transfusion tube, a silicone infusion tube, a dialysis membrane, a medical coating, or a shaping material integrated with a surface of the hollow fiber, the silicone blood transfusion tube, the silicone infusion tube, the dialysis membrane, or the medical coating.

10. A use of the micro-nano medical adsorbent resin powder material according to any one of claims 1, 2, 3, 4, 5 or 6, **characterized in that**: the powder material is used as a solute, and an injection solution or a physiological saline or ethanol having different purities, including fruit wine is used as a solvent to prepare a solution.

11. A use of the micro-nano medical adsorbent resin powder material according to any one of claims 1, 2, 3 4, 5 or 6, **characterized in that**: the powder material is added into a shaping material to make a paste, or a pill, or a grease agent.
